# EUROPEAN PATENT SPECIFICATION

(11) **EP 4 551 289 B1**
(45) Date of publication and mention of the grant of the patent: **23.09.2026**
(21) Application number: 23840159.0
(22) Date of filing: 08.07.2023
(51) Int. Cl.: A61N 1/05, A61N 1/39, A61N 1/00, A61N 1/04

(54) **DEFIBRILLATION SYSTEM HAVING DEFORMABLE PADDLE HEAD**
DEFIBRILLATIONSSYSTEM MIT VERFORMBAREM PADDELKOPF
SYSTÈME DE DÉFIBRILLATION AYANT UNE TÊTE D'ÉLECTRODE DÉFORMABLE

(30) Priority: 09.07.2022 US 202263359813 P; 07.07.2023 US 202318349121
(43) Date of publication of application: 14.05.2025
(73) Proprietor: USB Medical, Ltd., Hatboro, PA 19040 (US)
(72) Inventor: EPSTEIN, Stephen T., Newtown, Pennsylvania 18940 (US); SINISI, John J., Warminster, Pennsylvania 18974 (US); SINISI, Jesse, Warminster, Pennsylvania 18974 (US)
(74) Representative: MacLachlan IP Limited
(86) International application number: PCT/US2023/027182
(87) International publication number: WO 2024/015268

(56) References cited:
- WO-A1-2007/051648
- US-A- 5 904 711
- US-A1- 2003 191 501
- US-A1- 2003 191 501
- US-A1- 2014 155 968
- US-A1- 2018 036 528
- US-B1- 8 452 393
- US-B2- 10 682 511
- US-B2- 9 314 610

## Description

### TECHNICAL FIELD OF THE INVENTION

In general, the present disclosure relates to defibrillation paddles. More particularly, the present disclosure relates to defibrillation paddles that are used during open and minimally invasive procedures wherein the defibrillation paddle is brought into direct contact with the tissue of the heart.

### BACKGROUND ART

It is known that a heart muscle that has stopped beating or is beating erratically can sometimes be caused to beat normally by passing an electrical current through the tissue of the heart. The science of applying an electrical current to the heart is known as defibrillation and has been evolving for many years.

Defibrillator systems are specifically designed to pass an electrical current into a patient's heart. In the field of defibrillation, there are non-intrusive defibrillator paddles and intrusive defibrillator paddles. Non-intrusive defibrillator paddles have two electrodes that attach to the skin of a patient. The non-intrusive defibrillator paddles pass electricity through the body from one external point to another. Such non-intrusive defibrillator paddles are used by rescue workers, paramedics, and the like to revive a person whose heart has stopped.

Intrusive defibrillator paddles are used by physicians primarily in the operating room of hospitals. During many surgical procedures, a patient's heart may be temporarily stopped. In such situations, the patient's blood flow is transferred to an external pump. Once the surgical procedure is complete, a defibrillator paddle is commonly used to restart the heart. When the heart muscle itself is exposed, defibrillator paddles can be touched directly to the heart muscle. A small jolt of electricity is then passed through the tissue of the heart muscle to restart the heartbeat. Since the electricity is being applied directly to the heart muscle, low currents of electricity are used. However, even these low currents of electricity can result in some heart muscle tissue becoming burned in the areas of contact with the defibrillator paddles, especially if there is not good contact with the heart tissue.

One way to reduce the potential of damage to the heart muscle is to use a defibrillation system that touches the heart with only a single paddle. If only one paddle touches the heart, it is easier for a physician to bring that single paddle into proper contact with the heart.

In the prior art, defibrillator systems have been made that use only one paddle to contact the heart. In such systems, a patient is placed upon a conductive pad. A single paddle is provided. Both the single paddle and the conductive pad are connected to the same defibrillator system to create a circuit. The single paddle is then touched to the tissue of the heart. Electricity passes through the heart, through the back of the body and to the conductive pad. Since only one paddle is used, the chances that one paddle will be poorly positioned is reduced by half, as compared to a two paddle system. Such prior art defibrillation systems are exemplified by Japanese Reference No. JP2001121885, entitled Defibrillating Electrode And Defibrillation System; U.S. Pat. No. 8,452,393 to Epstein, entitled Defibrillation Paddle Structure And Its Associated Method Of Use, and U.S. Patent No. 10,682,511 to Epstein, entitled Defibrillator For Minimally Invasive Surgical Procedures.

Reference is made to US 2003/191501 A1, entitled "Sterile Disposable Internal Defibrillation Paddles", which discloses a disposable internal defibrillator containing: (a) a handle attached to a shaft attached to a spoon, the spoon having a receiving unit, and the handle, the shaft, and the spoon are all made of non conducting material; and (b) an electrode plate attaching onto the receiving unit of the spoon.

Reference is also made to US 2018/036528 A1, entitled "Defibrillator for minimally invasive surgical procedures", which discloses a defibrillation system wherein the paddle assembly has a paddle head with a conductive surface. The paddle head is introduced into the chest through a small surgical incision. The paddle head is connected to a handle by an elongated shaft. The elongated shaft has an electrically conductive core that is covered in a dielectric insulator. The elongated shaft is malleable. A pivot connection joins one end of the elongated shaft to the paddle head. The pivot connection enables the paddle head to move through a range of different orientations with respect to the elongated shaft. Electricity passes through the conductive core of the elongated shaft and into the paddle head.

When surgery is performed on the heart, the surgery can often be defined as either open heart surgery or minimally invasive surgery. During open heart surgery, the ribcage is opened at the sternum by the surgeon to expose the heart. This type of surgery requires a long and painful recovery period as the ribcage heals. As surgical procedures evolve, an increasing number of surgical procedures on the heart are being performed using minimally invasive techniques. When minimally invasive surgical techniques are used, the ribcage remains largely undisturbed. Small entrance holes, referred to as working ports, are formed between the ribs of the ribcage that enable elongated surgical instruments to be advanced toward the heart in the chest cavity. However, this presents certain problems, especially when it comes to defibrillation.

When the paddle head of a defibrillator system contacts the muscle tissue of the heart, the largest contact area possible is desired. If only a small contact area exists, all of the electrical current passing into the heart is forced to travel through the small area of tissue in contact with the defibrillator paddle head. This concentrates the electricity and can cause tissue burning and damage to the heart tissue. A large area of contact is desired because it distributes the flow of electrical current and is, therefore, less likely to cause burning. The key to avoiding tissue damage made by a defibrillator paddle is to have uniform contact between the paddle and the tissue of the heart muscle. To further complicate matters, a physician may decide to shock the heart at a specific spot. That location may be at the vertical apex, along the right ventricle or along the left ventricle. The doctor must maneuver the defibrillator paddle to that portion of the heart while still maintaining a flush contact between the defibrillator paddle and the tissue of the heart. If there is not a flush contact, the odds greatly increase that the defibrillator paddle may cause an electrical burn. Furthermore, without a flush contact, the defibrillator paddle may fail to affect the heart muscle in the desired manner.

A further problem exists because, in order to use a defibrillation paddle in a minimally invasive surgical technique, the paddle head must be small and of the proper shape and construction to reach the heart and work as intended. Current configurations of paddle heads have round shapes with complicated assemblies on the back side of the paddle head that take up valuable space. This makes such paddle heads expensive to manufacture and too large for most current minimally invasive procedures.

A need therefore exists for an improved defibrillation paddle design with an improved paddle shape that can be economically constructured, is more reliable than prior art paddles and functions better than prior art defibrillation paddles when used during mininimally invasive procedures. These needs are met by the present invention as decribed and claimed below.

### DISCLOSURE OF THE INVENTION

The present disclosure is a defibrillation system that utilizes an improved paddle assembly with an elliptical or oval shape and a uniquely simplified flexible construction. The paddle assembly has a paddle head that physically touches the heart during the defibrillation procedure. The paddle head is manipulated using a handle. The paddle head includes an enlarged support pad that is attached to the handle via a flexible neck. Both the enlarged support pad and the neck are formed from a thermoplastic elastomer and are highly flexible. Due to the flexibility of the material, both the enlarged support pad and the neck can be advanced through a small incision during a minimally invasive procedure.

A contact electrode is attached to the enlarged support pad. The contact electrode conducts electrical current to the heart. The enlarged support pad is thin and can deform with the enlarged support pad. The contact electrode receives electrical current through a conducive core element. The conductive core element extends through the handle and into the flexible paddle head, wherein the conductive core element is electrically coupled to the contact electrode via a terminal lead within the flexible paddle head.

The flexible neck is molded in a position where the flexible neck extends out of the side of the enlarged support pad. This low-profile configuration is an important advantage for minimally invasive procedures. In addition, the elliptical or oval shape of the enlarged support pad enables the overall paddle head to be narrower side to side while maintaining a larger contact area for proper electrical disbursement. In this manner, a contact area can be maintained that is equivalent to a rounder head configuration that would not fit through the same sized surgical entry point. This width advantage could be the deciding factor between selecting between a minimally Invasive approach and a far more invasive open approach that would result is a longer recovery time, larger scars, and more possibilities for infection.

### BRIEF DESCRIPTION OF THE DRAWINGS

For a better understanding of the present disclosure reference is made to the following description of an exemplary embodiment thereof, considered in conjunction with the accompanying drawings, in which:
FIG. 1 shows an exemplary embodiment of a defibrillation system;
FIG. 2 is an enlarged view of the paddle assembly used in the exemplary embodiment of Fig. 1;
FIG. 3 is an exploded perspective view of the paddle assembly shown in Fig. 2; and
FIG. 4 is an exploded and partially cross-sectioned side view of the paddle assembly used in Fig. 2.

### DETAILED DESCRIPTION OF BEST MODE FOR CARRYING OUT THE INVENTION

The present invention is defined by the appended claims. Aspects, embodiments and examples described hereinafter are only of exemplary nature and are presented for better understanding the present invention. Although the present disclosure defibrillation paddle can be embodied in many ways, the embodiment illustrated shows only one paddle design. The embodiment is selected in order to set forth one of the best modes contemplated for the disclosure

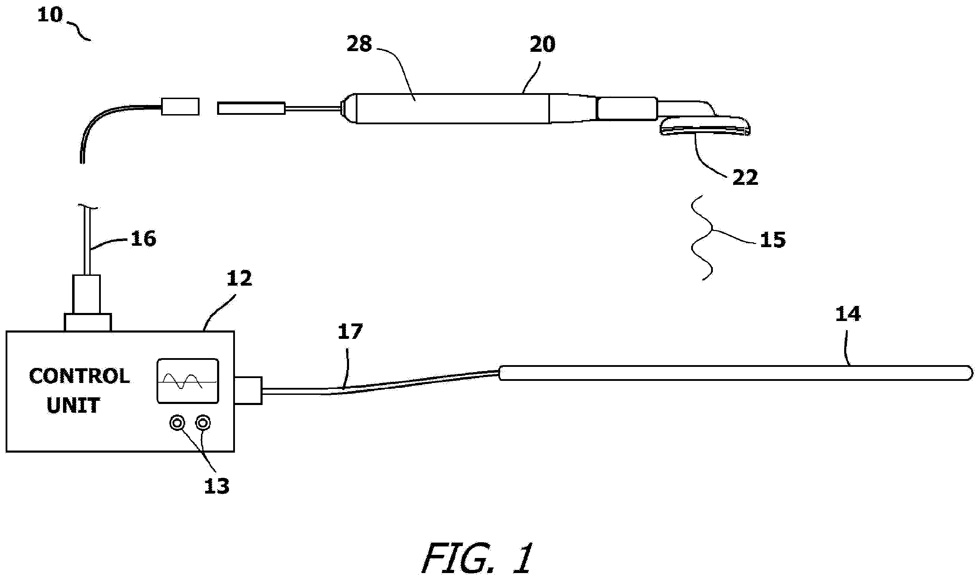

Referring to Fig. 1, there is shown an exemplary embodiment of a defibrillation system 10 in accordance with the presentdisclosure. The defibrillation system 10 consists of a control unit 12 that controls the electrical current and waveform that will be passed through the heart muscle during the defibrillation procedure. The control unit 12 has adjustable controls 13 and safeguards common in the industry. There are several defibrillation systems that are commercially available. The control unit of most prior art defibrillation systems can be adapted for use as part of the present disclosure defibrillation system 10.

The control unit 12 creates the waveform 15 that is applied to the heart. To apply the waveform 15 to the heart, an electrical pathway must be created that passes through the heart. To create the needed electrical pathway, a conductive pad 14 is provided. The conductive pad 14 is placed under a prone patient in a position that is below the heart in the vertical plane. The conductive pad 14 contacts the skin of the patient across a wide contact area. The conductive pad 14 is connected to the control unit 12 by a first flexible terminal wire 17.

The waveform 15 from the control unit 12 is applied to the patient using a paddle assembly 20. The paddle assembly 20 can be selectively attached to the control unit 12 via the second flexible terminal wire 16. The paddle assembly 20 has a contact electrode 22. The control unit 12 creates an electrical bias between the contact electrode 22 on the paddle assembly 20 and the conductive pad 14. Accordingly, when both the conductive pad 14 and the contact electrode 22 of the paddle assembly 20 touch a patient, current waveform 15 can flow through the patient between these surfaces.

In practice, the conductive pad 14 is externally placed under a prone patient. As part of a minimally invasive procedure, the paddle assembly 20 is manipulated by a surgeon inside the body so that the contact electrode 22 contacts the heart in a particular place. The defibrillation system 10 is then activated so that the current waveform 15 passes into the paddle assembly 20, through the contact electrode 22, through the heart, and through the portion of the patient's body between the heart and the conductive pad 14.

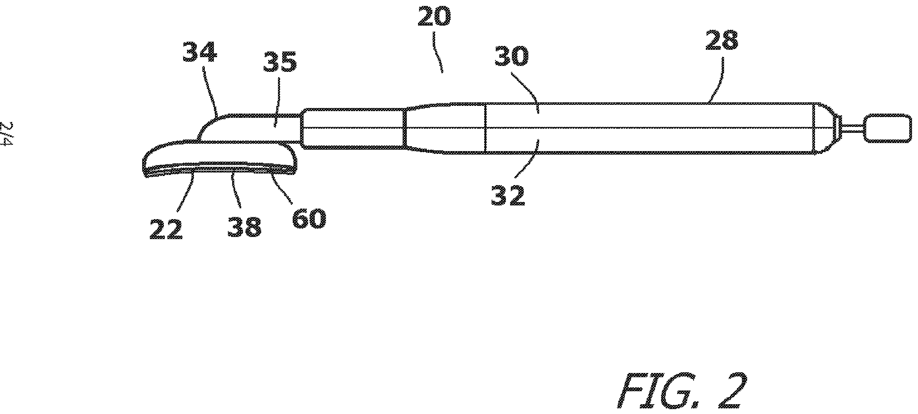

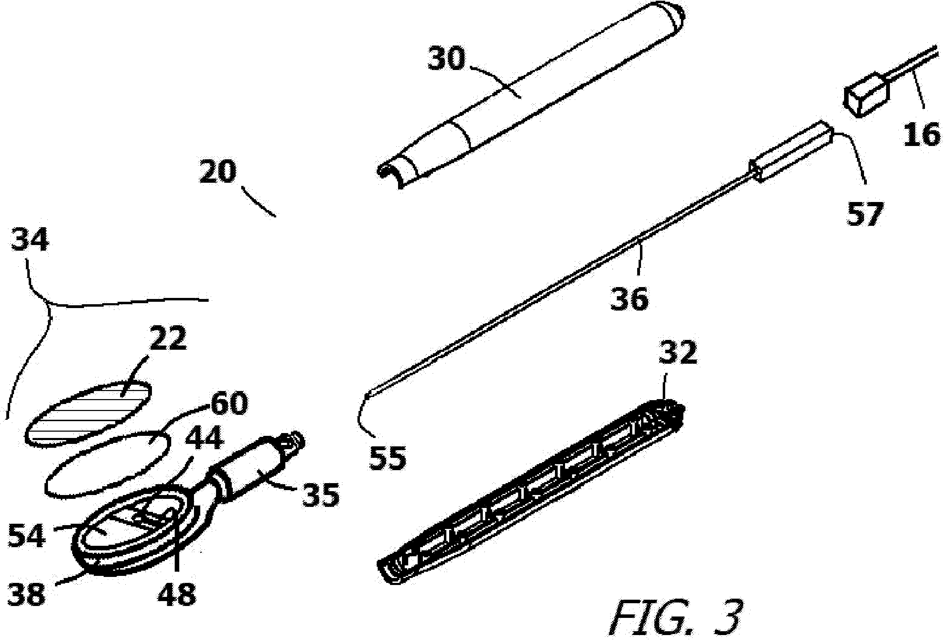

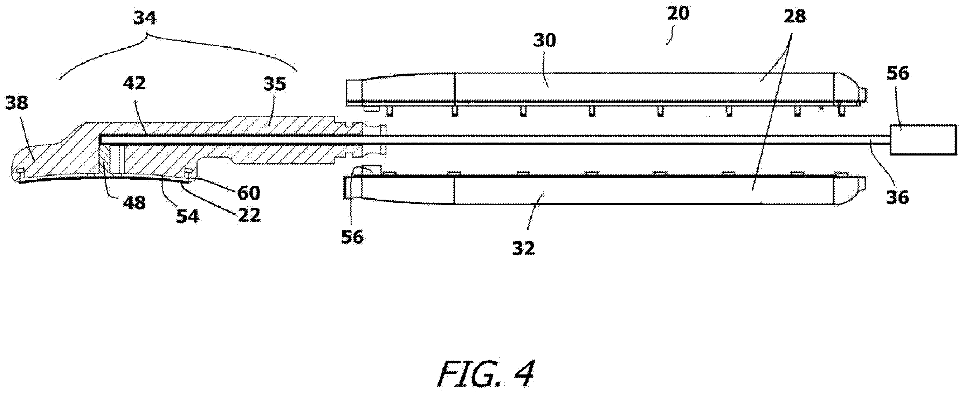

Referring to Fig. 2, Fig. 3 and Fig. 4, the details for the structure of the paddle assembly 20 are shown. The paddle assembly 20 has handle 28 to facilitate the manual manipulation of the paddle assembly 20. The handle 28 can be straight, or it can be ergonomically shaped to fit comfortably in a physician's hand. The handle 28 can have many lengths, wherein a physician selects the handle length depending upon the size of the patient and the intricacies of the procedure. In the shown embodiment, the paddle assembly 20 is one that can be used in either a minimally invasive procedure or an open invasive procedure. The paddle assembly 20 and has a handle 28 that will not block access of other instruments through a small incision. It will be understood that the handle 28 can be larger if used in open chest procedures.

The handle 28 has a two-part clamshell design, wherein the handle 28 has two halves 30, 32. The two halves 30, 32 are molded from a robust plastic and are textured to prevent a grip from slipping. When assembled, the handle 28 has a first end 29 and an opposite second end 31. The two halves 30, 32 are designed to engage and retain both the paddle assembly 20 and a conductive core element 36. The handle 28 supports the paddle assembly 20 so that the paddle assembly 20 extends from the first end 29 of the handle 28. The conductive core element 36 extends through the handle 28 from the paddle assembly 20 to the second end 31 of the handle 28.

The paddle assembly 20 includes a paddle head 34 that is disposed at the end of a flexible neck 35. The flexible neck 35 is concentrically aligned with the handle 28 and extends into the first end 29 of the handle 28. The low cost one-piece flexible neck 35 acts as a cantilever and self-centering spring that supports the paddle head 34 and attaches the paddle head but also allows it to be flexed into any position around the heart.

The paddle head 34 contains an enlarged support pad 38 that is generally circular and optimally elliptical in shape. The flexible neck 35 extends from the peripheral side of the enlarged support pad 38. In this manner, the presence of the flexible neck 35 does increase the thickness of the enlarged support pad 38 or the overall thickness of the paddle head 34. Both the enlarged support pad 38 and the flexible neck 35 are unistucturally molded from a curable silicone or another soft thermoplastic elastomer. Accordingly, depending upon the flexibility of the contact electrode 22 being supported, the enlarged support pad 38 can be deformed by applying sufficient forces to the structure. If the contact electrode 22 is relatively stiff, small deflections can be made that will assist the paddle head 34 in passing into an incision. If the contact electrode 22 is a highly flexible foil or similar conductive surface, then the enlarged support pad 38 can be rolled into a size just wider than that of the flexible neck 35. In this manner, the entire paddle assembly 20, up to the handle 28, can be advanced through a narrow incision that is only slightly larger than the diameter of the flexible neck 35. Likewise, the flexible neck 35 can be bent using the application of external forces. The ability of the enlarged support pad 38 and the flexible neck 35 to bend and deform is very useful in inserting the overall paddle assembly 20 through a small surgical incision and positioning the paddle head 34 properly in relation to the heart during a surgical procedure.

An open conduit 42 extends through the flexible neck 35 and into the enlarged support pad 38. The open conduit 42 is sized to receive a section of the conductive core element 36. A traverse hole 44 is formed in the enlarged support pad 38 at or near its center. The traverse hole 44 intersects the open conduit 42 at a perpendicular. A conductive contact terminal 48 extends through the traverse hole 44. The conductive contact terminal 48 intersects the open conduit 42. The opposite end of the conductive contact terminal 48 terminates at a face surface 54 of the enlarged support pad 38. Accordingly, the conductive contact terminal 48 connects the open conduit 42 to the face surface 54 of the paddle head 34.

The conductive core element 36 extends into the open conduit 42 of the paddle head 34. The conductive core element 36 has a first end 55 and an opposite second end 57. The first end 55 of the conductive core element 36 contacts, and electrically interconnects, with the contact terminal 48 that extends through the traverse hole 44. The second end 57 of the conductive core element 36 extends out of the open conduit 42 in the paddle head 34 and through the handle 28. The second end 57 of the conductive core element 36 terminates with a connector plug 58 beyond the second end 31 of the handle 28. The connector plug 58 attaches to the second terminal wire 16 of the overall defibrillation system 10.

Within the handle 28 is a connector 56. The connector 56 engages the conductive core element 36 as the two halves 30, 32 of the handle 28 are joined around the conductive contact terminal 48. The connector 56 locks the conductive core element 36 in a fixed position relative to the handle 28.

In the handle 28, the conductive core element 36 is locked into a fixed position. However, the conductive core element 36 also extends through the flexible neck 35. The conductive core element 36 itself is also flexible. In this manner, when the paddle head 34 is distorted, the conductive core element 36 can bend within the flexible neck 35 to be better positioned against the heart.

Within the paddle head 34, the conductive core element 36 terminates at the conductive contact terminal 48, wherein both the conductive core element 36 and the conductive contact terminal 48 electrically interconnect. The conductive contact terminal 48 extends through the support pad 38 to the face surface 54 of the paddle head 34. The conductive contact terminal 48 electrically connects to a conductive support 60. The conductive support 60 can either be a removable element or can be molded into the paddle head 34. The conductive support 60 provides direct support to the contact electrode 22. The conductive support 60 is generally the same size as the contact electrode 22. In this manner, the full contact electrode 22 will receive an evenly distributed charge from the conductive support 60. It is the contact electrode 22 that physically contacts the heart.

Referring to all figures, it will now be understood that to utilize the defibrillation system 10, the conductive pad 14 is placed under a patient. Using existing access incisions or a newly formed access incision, the paddle head 34 of the overall paddle assembly 20 is advanced in vivo to the heart. Using the handle 28, a physician positions the paddle head 34 against the heart. The paddle head 34 is flexible and can conform to the contours of the heart. This places the contact electrode 22 flush against the heart. In this manner, the current waveform passing into the heart is evenly distributed across the full area of the contact electrode 22, therein preventing electrical burn damage to the heart.

Most of the paddle head 34 is molded from a thermoplastic elastomer. The conductive elements within the paddle head 34 contain only a small amount of conductive metal. The handle 28 is also a low-cost molded construct. The most expensive component in terms of fabrication materials is the conductive core element, which is nothing more than a short length of flexible wire. Accordingly, the whole of the paddle assembly 20 can be made near the cost of cleaning, sterilizing, and repackaging a reusable defibrillation paddle. The result is a defibrillation paddle assembly 10 that can be considered disposable and economical for one-time-use.

## Claims

1. A defibrillation paddle assembly (20), comprising:
a flexible paddle head (34) having
a flexible neck (35) unistructurally molded from a curable silicone elastomeric material, wherein the flexible paddle head (34) comprises a support pad (38);
a contact electrode (22) mounted on said support pad (38);
a handle (28) coupled to said flexible paddle head (34) for selectively manipulating said flexible paddle head (34), wherein said handle (28) has a two-part clamshell design that includes a first half (30) and a second half (32), wherein said first half (30) and said second half (30) of said handle close around and engage said flexible neck (35), wherein said flexible neck (35) extends concentrically from said handle (28) and acts as a cantilever and self-centering spring in supporting said flexible paddle head (34) at a first distance from said handle (28);
a conductive core element (36) having a first end (55) and an opposite second end (57), wherein said conductive core element (36) extends through said handle (28) and through said flexible neck (35), wherein said first end (55) of said conductive core element (36) terminates in said flexible paddle head (34) and said second end (57) of said conductive core element (36) terminates outside said handle (28), wherein said first end (55) of said conductive core element(36) is electrically coupled to said contact electrode (22) within said flexible paddle head (34); and
a connector (56) disposed within said handle (28) that engages said conductive core element (36) and locks said conductive core element (36) in a fixed position relative to the handle (28) as said first half (30) and said second half (32) of the handle (28) are joined.

2. The assembly according to Claim 1, wherein said support pad (38) has a width that can be reduced by elastic deformation of said support pad (38).

3. The assembly according to Claim 1, wherein said conductive core element (36) is flexible.

4. The assembly according to Claim 3, wherein said second end (57) of said conductive core element (36) terminates with a wire connector (56) outside said handle (28).

5. The assembly according to Claim 1, further including a terminal (48) that electrically interconnects said contact electrode (22) to said conductive core element (36) within said flexible paddle head (34).

6. The assembly according to Claim 1, wherein said handle (28) engages and retains said conductive core element (36) and prevents relative movement of said conductive core element (36) within said handle (28).

7. The assembly according to Claim 1, wherein said support pad is a wide support pad (38) and said flexible neck is a narrower neck (35), wherein said conductive core element (36) extends into said support pad (38) through said neck (35); optionally
wherein said support pad (38) has a width and can be elastically deformed into a smaller width for insertion into a person's body; optionally
wherein said conductive core element (36) is flexible; and optionally
wherein said second end (57) of said conductive core element (36) terminates with a wire connector (56) outside of said handle (28).

## Patentansprüche

1. Defibrillationspaddelanordnung (20), umfassend:
einen flexiblen Paddelkopf (34) mit einem flexiblen Hals (35), der einstückig aus einem härtbaren Silikonelastomermaterial geformt ist, wobei der flexible Paddelkopf (34) eine Stützunterlage (38) umfasst;
eine Kontaktelektrode (22), die auf der Stützunterlage (38) montiert ist;
einen Griff (28), der an den flexiblen Paddelkopf (34) gekoppelt ist, zum selektiven Manipulieren des flexiblen Paddelkopfs (34), wobei der Griff (28) eine zweiteilige Muschelschalenkonstruktion aufweist, die eine erste Hälfte (30) und eine zweite Hälfte (32) beinhaltet, wobei die erste Hälfte (30) und die zweite Hälfte (30) des Griffs um den flexiblen Hals (35) herum schließen und diesen in Eingriff nehmen, wobei sich der flexible Hals (35) konzentrisch von dem Griff (28) erstreckt und als eine freitragende und selbstzentrierende Feder beim Stützen des flexiblen Paddelkopfs (34) in einem ersten Abstand von dem Griff (28) wirkt;
ein leitfähiges Kernelement (36) mit einem ersten Ende (55) und einem gegenüberliegenden zweiten Ende (57), wobei sich das leitfähige Kernelement (36) durch den Griff (28) und durch den flexiblen Hals (35) erstreckt, wobei das erste Ende (55) des leitfähigen Kernelements (36) in dem flexiblen Paddelkopf (34) endet und das zweite Ende (57) des leitfähigen Kernelements (36) außerhalb des Griffs (28) endet, wobei das erste Ende (55) des leitfähigen Kernelements (36) elektrisch an die Kontaktelektrode (22) innerhalb des flexiblen Paddelkopfs (34) gekoppelt ist; und
einen Verbinder (56), der innerhalb des Griffs (28) angeordnet ist, der das leitfähige Kernelement (36) in Eingriff nimmt und das leitfähige Kernelement (36) in einer festen Position relativ zu dem Griff (28) verriegelt, wenn die erste Hälfte (30) und die zweite Hälfte (32) des Griffs (28) miteinander zusammengefügt sind.

2. Anordnung nach Anspruch 1, wobei die Stützunterlage (38) eine Breite aufweist, die durch elastische Verformung der Stützunterlage (38) reduziert werden kann.

3. Anordnung nach Anspruch 1, wobei das leitfähige Kernelement (36) flexibel ist.

4. Anordnung nach Anspruch 3, wobei das zweite Ende (57) des leitfähigen Kernelements (36) mit einem Drahtverbinder (56) außerhalb des Griffs (28) endet.

5. Anordnung nach Anspruch 1, ferner beinhaltend einen Anschluss (48), der die Kontaktelektrode (22) elektrisch mit dem leitfähigen Kernelement (36) innerhalb des flexiblen Paddelkopfs (34) verbindet.

6. Anordnung nach Anspruch 1, wobei der Griff (28) das leitfähige Kernelement (36) in Eingriff nimmt und dieses hält und eine Relativbewegung des leitfähigen Kernelements (36) innerhalb des Griffs (28) verhindert.

7. Anordnung nach Anspruch 1, wobei die Stützunterlage eine breite Stützunterlage (38) ist und der flexible Hals ein schmalerer Hals (35) ist, wobei sich das leitfähige Kernelement (36) durch den Hals (35) in die Stützunterlage (38) erstreckt; optional,
wobei die Stützunterlage (38) eine Breite aufweist und zum Einführen in den Körper einer Person elastisch zu einer kleineren Breite verformt werden kann; optional,
wobei das leitfähige Kernelement (36) flexibel ist; und optional,
wobei das zweite Ende (57) des leitfähigen Kernelements (36) mit einem Drahtverbinder (56) außerhalb des Griffs (28) endet.

## Revendications

1. Ensemble électrode de défibrillation (20), comprenant :
une tête d'électrode souple (34) ayant un col souple (35) moulé de manière unitaire à partir d'un matériau élastomère de silicone durcissable, dans lequel la tête d'électrode souple (34) comprend un coussinet de support (38) ;
une électrode de contact (22) montée sur ledit coussinet de support (38) ;
une poignée (28) couplée à ladite tête d'électrode souple (34) pour manipuler sélectivement ladite tête d'électrode souple (34), dans lequel ladite poignée (28) a une conception à clapet en deux parties qui comprend une première moitié (30) et une seconde moitié (32), dans lequel ladite première moitié (30) et ladite seconde moitié (30) de ladite poignée se referment sur ledit col souple (35) et viennent en prise avec celui-ci, dans lequel ledit col souple (35) s'étend concentriquement à partir de ladite poignée (28) et agit comme un ressort en porte-à-faux et autocentré en supportant ladite tête d'électrode souple (34) à une première distance de ladite poignée (28) ;
un élément central conducteur (36) ayant une première extrémité (55) et une seconde extrémité opposée (57), dans lequel ledit élément central conducteur (36) s'étend à travers ladite poignée (28) et à travers ledit col souple (35), dans lequel ladite première extrémité (55) dudit élément central conducteur (36) se termine dans ladite tête d'électrode souple (34) et ladite seconde extrémité (57) dudit élément central conducteur (36) se termine à l'extérieur de ladite poignée (28), dans lequel ladite première extrémité (55) dudit élément central conducteur (36) est électriquement couplée à ladite électrode de contact (22) au sein de ladite tête d'électrode souple (34) ; et
un raccord (56) disposé au sein de ladite poignée (28) qui vient en prise avec ledit élément central conducteur (36) et verrouille ledit élément central conducteur (36) dans une position fixe par rapport à la poignée (28) lorsque ladite première moitié (30) et ladite seconde moitié (32) de la poignée (28) sont jointes.

2. Ensemble selon la revendication 1, dans lequel ledit coussinet de support (38) a une largeur qui peut être réduite par déformation élastique dudit coussinet de support (38).

3. Ensemble selon la revendication 1, dans lequel ledit élément central conducteur (36) est souple.

4. Ensemble selon la revendication 3, dans lequel ladite seconde extrémité (57) dudit élément central conducteur (36) se termine par un raccord de câble (56) à l'extérieur de ladite poignée (28).

5. Ensemble selon la revendication 1, comprenant en outre une borne (48) qui relie électriquement ladite électrode de contact (22) audit élément central conducteur (36) au sein de ladite tête d'électrode souple (34).

6. Ensemble selon la revendication 1, dans lequel ladite poignée (28) vient en prise avec ledit élément central conducteur (36) et retient celui-ci, et empêche un mouvement relatif dudit élément central conducteur (36) au sein de ladite poignée (28).

7. Ensemble selon la revendication 1, dans lequel ledit coussinet de support est un coussinet de support large (38) et ledit col souple est un col plus étroit (35),
dans lequel ledit élément central conducteur (36) s'étend dans ledit coussinet de support (38) à travers ledit col (35) ; éventuellement dans lequel ledit coussinet de support (38) a une largeur et peut être déformé élastiquement en une largeur plus petite pour être inséré dans le corps d'une personne ; éventuellement
dans lequel ledit élément central conducteur (36) est souple ; et éventuellement
dans lequel ladite seconde extrémité (57) dudit élément central conducteur (36) se termine par un raccord de câble (56) à l'extérieur de ladite poignée (28).
